# EUROPEAN PATENT APPLICATION

(11) **EP 4 623 982 A1**
(43) Date of publication of application: **01.10.2025**
(21) Application number: 24167927.3
(22) Date of filing: 29.03.2024
(51) Int. Cl.: A61M 37/00

(54) **MICRONEEDLE DEVICE**

(71) Applicant: 3C Project Technologies Limited, Greystones 281603 (IE); University College Cork - National University of Ireland, Cork, Cork (IE)
(72) Inventor: MCAVOY, Gregor John, County Wicklow, 281603 Greystones (IE); RICE, Henry, County Wicklow, 281603 Greystones (IE); O'MAHONY, Conor, Cork, T12 K8AF (IE); WEBSTER, Carlo, Cork, T12 K8AF (IE)
(74) Representative: Hindles Limited

(57) **Abstract**

A microneedle device 100 having one or more microneedles 106 extending from a substrate 104, and for insertion into a target body. The microneedle device includes one or more piezoelectric micromachined ultrasound transducer 102, PMUT, elements formed on the substrate. Each of the one or more PMUT elements includes a moveable piezoelectric diaphragm. Control circuitry 110 is integrally formed with the substrate and electrically coupled to the one or more PMUT elements.

## Description

### Field of the invention

The present invention relates to microneedle devices.

### Background to the invention

Microneedle arrays are useful for a range of biomedical applications, such as transdermal drug delivery and fluid extraction. They can provide a less invasive way of delivering or extracting fluid compared to conventional hypodermic needles. They are also known for use in veterinary, cosmetic and agricultural applications.

Microneedle arrays include an arrangement of micron sized needles (typically around 50-1500 µm in length, 50-250 µm in width, and 1-25 µm in diameter). Various types of microneedles are known. For example, they may be solid, dissolvable, hollow and/or coated. Silicon is a commonly used material - but other options such as metals, ceramics, and polymers are also possible.

It is known to provide microneedle arrays coupled to an ultrasound transducer for drug delivery purposes. However, providing devices which are portable whilst also being convenient and cost-effective to manufacture is challenging.

It is in this context that the present invention has been devised.

### Summary of the invention

According to an aspect of the invention, there is provided a microneedle device comprising:
a substrate;
one or more microneedles extending from the substrate, and for insertion into a target body;
one or more piezoelectric micromachined ultrasound transducer, PMUT, elements formed on the substrate, wherein each of the one or more PMUT elements comprises a moveable piezoelectric diaphragm;
and control circuitry integrally formed with the substrate and electrically coupled to the one or more PMUT elements.

By providing one or more microneedles extending from a substrate having at least one PMUT element formed thereon and having integrated control circuitry, it is possible to provide a highly integrated microneedle device having a small-form factor. This arrangement avoids or limits the need for separate electronics (not integrated with the control circuitry). Accordingly, it is made possible to provide microneedle devices which are portable whilst also having the high functionality (e.g., control/sensing) enabled by the combination of a PMUT element and control circuitry.

Since each PMUT element is formed on the substrate having the integrally formed control circuitry, the device is also relatively straightforward to manufacture (e.g., compared to other integrated devices where the MEMS components and control circuitry are manufactured separately before being bonded together).

The substrate may comprise a silicon wafer. The substrate may be a semiconductor substrate.

An additional layer may be provided between the microneedle and the substrate - e.g., a valve seat/other sealing layer.

The term microneedle device will be understood to mean substantially any device having at least one micro-sized needle (e.g., 50-1500 µm in length, 50-250 µm in width, and 1-25 µm in diameter) for insertion into a target body.

Typically, the moveable piezoelectric diaphragm comprises a piezoelectric body, a first electrode and a second electrode. The piezoelectric body, a first electrode and a second electrode are typically stacked on a first surface of the substrate (e.g., between the substrate and at least one of the plurality of microneedles).

Typically, the moveable piezoelectric diaphragm defines at least part of a wall of a cavity. Typically, the cavity is defined in part by the moveable piezoelectric diaphragm and in part by the substrate.

Each PMUT element is formed on (e.g., deposited on) the substrate. The PMUT element may be deposited using one or more PVD methods. Importantly, the PMUT element is formed on the substrate rather than being formed separately and later bonded.

It may be that the control circuity is configured to control reception of signals resulting from the movement of the moveable piezoelectric diaphragm. It may be that the control circuitry is configured to control movement of the moveable piezoelectric diaphragm.

The microneedle device may be a microneedle array. The term microneedle array will be understood to mean substantially any device having an arrangement of micro-sized needles (e.g., 50-1500 µm in length, 50-250 µm in width, and 1-25 µm in diameter) for insertion into a target body.

It may be that there is uniform spacing between adjacent microneedles of the microneedle device.

It may be that the control circuity comprises CMOS control circuitry.

The CMOS control circuitry may be formed using conventional CMOS fabrication techniques (e.g. ion implantation, chemical vapour deposition (CVD), physical vapour deposition (PVD), etching, chemical-mechanical planarization (CMP) and/or electroplating).

It may be that each of the one or more PMUT elements is coupled to at least one microneedle of the one or more microneedles. It may be that each microneedle is coupled to a single PMUT element. It may be that each PMUT element is coupled to a corresponding single microneedle.

The response of the PMUT element (i.e., the response of the piezoelectric diaphragm of that PMUT element) affects the microneedle coupled therewith (and vice versa).

In some embodiments, each microneedle is coupled to a corresponding single PMUT element and each PMUT element is coupled to a corresponding single microneedle (i.e., there is a one-to-one correspondence between microneedles and PMUT elements).

By providing control circuitry integrated with the PMUT elements as described herein, it is possible to provide a high number of microneedles coupled to PMUT elements on a single substrate, whilst enabling functionality on the scale of individual microneedles.

It may be that the control circuity is configured to receive electrical signals generated by the movement of the or each moveable piezoelectric diaphragm, and generate a signal based on the movement of the or each moveable piezoelectric diaphragm.

By providing control circuitry configured to receive electrical signals generated by each PMUT element, it is possible to provide a microneedle device having integrated sensing capabilities. This in advantageous for a range of applications, as described herein.

It may be that the microneedle device further comprises a first circuit, wherein the first circuit is configured to determine a microneedle characteristic based on the signal. It may be that the microneedle characteristic is indicative of whether at least one of the one or more microneedles is correctly inserted in the target body. It may be that the first circuit is configured to determine a target body characteristic based on the signal. It may be that the target body characteristic is indicative of an internal pressure of the target body.

The inventors have realised that it is possible to use the sensed data to obtain characteristics of microneedles of the device, thereby facilitating improved device operation/performance.

It may be that the microneedle device is configured such that characteristics are determinable on the scale of individual microneedles/PMUT elements.

It may be that a first PMUT element is coupled to a first microneedle, wherein the first circuit is configured to determine a microneedle characteristic of the first microneedle based on the movement of the moveable piezoelectric diaphragm of the first PMUT element.

It may be that the microneedle characteristic and/or the target body characteristic is determined in dependence on an oscillation of at least one of the moveable piezoelectric diaphragms. It may be that the microneedle characteristic and/or the target body characteristic is determined in dependence on a frequency of the oscillation.

The inventors have realised that the oscillations of the moveable piezoelectric diaphragms (including the frequency content of those oscillations) can provide an indication of characteristics of a microneedle and/or target body.

It will be understood that the signal comprises information indicative of the oscillation of at least one of the moveable piezoelectric diaphragms. The information indicative of the oscillation may be at least one of an amplitude and a frequency of the oscillation.

The information may be indicative of the frequency of the oscillation. The information may be the frequency of the oscillation.

It may be that the first circuit is configured to generate an output signal based on the determined microneedle characteristic and/or target body characteristic. It may be that the output signal is for disabling at least one of the one or more microneedles. It may be that the output signal is for alerting the user that operation of the microneedle device is compromised.

Advantageously, the microneedle device may be configured to respond to the determined characteristic to disable a microneedle or alert the user that the operation of the microneedle device is compromised (e.g., a microneedle is blocked, or not correctly inserted in the target body), thereby facilitating more efficient and effective use of the microneedle device.

The microneedle device may be configured to extract liquid from the target body. The microneedle device may be configured to deliver liquid to the target body.

The fluid may be extracellular fluid (e.g., interstitial skin fluid). The liquid may be a pharmaceutical fluid (e.g., medication/medicinal drugs such as antibiotics, insulin or vaccines). The fluid may comprise cannabinoids (e.g., cannabidiol). The fluid may comprise small molecule ingredients.

By providing a microneedle device configured to extract/deliver liquid to a target body, convenient and cost-effective testing/treatment of the target body is enabled. A microneedle device for delivering/extracting liquid having a small form factor is particularly advantageous in settings where providing a less bulky device is important (e.g., for improved patient comfort/adherence in a medical setting or when testing/treating a small target body).

It may be that the microneedle device comprises one or more chambers for accommodating at least a portion of the fluid between the one or more microneedles and the one or more PMUT elements (e.g., the one or more moveable piezoelectric diaphragms of the one or more PMUT elements) such that a force can be transferred between the or each PMUT element and the or each microneedle through the fluid (e.g., directly through the fluid). It may be that ultrasound can be transmitted between the one or more microneedles and the one or more PMUT elements through the fluid (e.g., directly through the fluid). It may be that the one or more chambers are in fluidic communication with the one or more microneedles.

It may be that each microneedle is coupled to a corresponding single PMUT element and each PMUT element is coupled to a corresponding single microneedle via a respective chamber for accommodating the fluid.

It may be that the microneedle device is configured for use with a specific type of fluid. For example, it may be that the microneedle device is configured to preserve the integrity (e.g., to avoid degradation/denaturation) of the specific type of fluid (e.g., a specific type of pharmaceutical fluid) being delivered and/or extracted. For example, the or each microneedle may be sized and/or shaped to preserve the integrity of the fluid. It may be that the movement of the or each moveable piezoelectric diaphragm is controlled to preserve the integrity of the fluid - e.g., suitable amplitudes/frequencies of oscillation are chosen. Thus, the forces exerted on the fluid are reduced (relative to conventional microneedle arrays). Advantageously, it is possible to provide fluid delivery/extraction with high efficacy and efficiency.

The microneedle device can be used for medical applications in a domestic or clinical setting, by trained medical professionals, or by the patient themselves. The microneedle device may be used for veterinary applications. The microneedle device may be used in an agricultural setting.

The target body may be part of a plant. The target body may be part of a human body. The target body may be part of an animal body.

In some embodiments, the or each moveable piezoelectric diaphragm defines at least part of a wall of a respective cavity, the microneedle device further comprises an input for receiving the liquid into the cavity. The input may in fluidic communication with a channel - e.g., a channel extending parallel to the moveable piezoelectric diaphragm (when the moveable piezoelectric diaphragm is not in an actuated configuration). It may be that the input receives the liquid into the cavity via a valve.

Advantageously, the inclusion of valves enhances the extraction/delivery of liquid.

It some embodiments, the microneedle device further comprises an input for receiving the liquid above the cavity (and respective moveable piezoelectric diaphragm). The input may in fluidic communication with a channel extending substantially parallel to moveable piezoelectric diaphragm (when the moveable piezoelectric diaphragm is not in an actuated configuration).

In some embodiments, the or each moveable piezoelectric diaphragm comprises an opening for fluidic connection between the respective cavity and at least one of the plurality of microneedles. It may be that the opening is arranged at the periphery of the or each respective moveable piezoelectric diaphragm.

In some embodiments, the microneedle device further comprises the liquid within the cavity.

It may be that the microneedle device is configured so that, in use, when the or each moveable piezoelectric diaphragm is deformed in a first sense, the respective cavity is in fluidic communication with at least one microneedle of the one or more microneedles, and wherein the or each moveable piezoelectric diaphragm is deformed in a second sense, the respective cavity is not in fluidic communication with any of the one or more microneedles.

It may be that the control circuitry is configured to sense fluid characteristics. For example, it may be that the control circuitry is configured to sense fluid flow rates through the device and/or fluid viscosity. It may be that the control circuitry is configured to sense whether the device contains fluid.

It may be that each microneedle defines at least in part a respective fluid channel extending therethrough, wherein the microneedle characteristic is indicative of whether a bubble exists within the fluid channel of a respective microneedle. It may be that the first circuit is configured to generate an output signal based on the microneedle characteristic. It may be that the output signal is for triggering removal of the bubble. It may be that the output signal is for disabling a microneedle. It may be that the output signal is for alerting the user that operation of the microneedle device is compromised.

Bubbles within the microneedles can affect the operation of the microneedle device - e.g., by preventing fluid delivery/extraction. Advantageously, the first circuit can be configured to generate a signal (e.g., to trigger an alert, or a control strategy) in response to the detection of such bubbles, thereby facilitating more efficient and effective use of the microneedle device. The first circuit may be further configured to trigger removal of the bubble, thereby improving the operation of the microneedle device by facilitating improved fluid flow through the microneedle channel.

The signal may trigger the generation of ultrasound to remove the bubble. For example, the signal may trigger movement of the moveable piezoelectric diaphragm of the PMUT element coupled to the microneedle having the bubble in the respective fluid channel.

It may be that the control circuitry comprises the first circuit.

By providing the first circuit within the integrated circuitry, the overall size of the microneedle device can be reduced (e.g., compared to embodiments where the first circuit is not provided within the substrate), thereby facilitating more portable microneedle devices.

It may be that the control circuitry is configured to control ultrasound transmission by controlling movement of the or each moveable piezoelectric diaphragm. It may be that the control circuitry is configured to control ultrasound reception by receiving signals resulting from movement of the or each moveable piezoelectric diaphragm.

Transmitted ultrasound can be used to improve fluid extraction and/or delivery, thereby providing a more effective microneedle device. In particular, the characteristics of transmitted ultrasound may be chosen to enhance fluid delivery through the user's skin. Without wishing to be bound by theory, this may involve increasing the permeability of the skin by disrupting cells/opening microscopic channels.

Received ultrasound can be used to detect microneedle characteristics (e.g., blockages) or characteristics of the target body (e.g., heart rate or pressure within the skin).

It may be that the control circuitry is configured to control ultrasound transmission.

It may be that the control circuitry is configured to control movement of the or each moveable piezoelectric diaphragm to extract liquid from the target body. It may be that the control circuitry is configured to control movement of the or each moveable piezoelectric diaphragm to deliver liquid to the target body.

The inventors have further realised that PMUT elements can be used to extract and/or deliver fluid to the target body. That is, the PMUT elements can effectively be used as pumps. It is possible for individual microneedles to have a dedicated pump provided by a respective PMUT element, thereby providing improved control of fluid delivery/extraction.

Typically, microneedle devices require external (i.e., non-integrated) pumps. By providing a microneedle device with integrated PMUT elements and the associated control circuitry, it is possible to provide more portable fluid delivery/extraction systems.

It may be that the microneedle device further comprises a temperature sensor for measuring the liquid temperature, wherein the control circuitry is configured to control movement of the or each moveable piezoelectric diaphragm based on the liquid temperature.

It will be understood that liquid viscosity is generally temperature dependent. By providing integrated temperature sensors and control circuitry configured to control the movement piezoelectric diaphragms based on the measured temperatures, improved fluid delivery/extraction is facilitated.

It may be that microneedle device further comprises a reservoir for storing a liquid therein. It may be that the reservoir contains a liquid for delivery to the target body and/or a liquid extracted from the target body.

It may be that the substrate has a first surface, the one or more microneedles extending from the first surface and the one or more PMUT elements formed on the first surface, wherein at least a portion of the control circuitry is integrally formed at the first surface.

By providing a microneedle device having one or more microneedles, one or more PMUT elements and control circuitry formed on the same surface the form factor of the device is further reduced.

It may be that the one or more PMUT elements each comprise one or more materials processable at a temperature below 450°C. It may be that the or each moveable piezoelectric diaphragm comprises scandium aluminium nitride.

Typically, each moveable piezoelectric diaphragm comprises a piezoelectric body, a first electrode and a second electrode. It may be that the piezoelectric body comprises one or more piezoelectric materials processable at a temperature below 450°C. Examples of piezoelectric materials that are processable at temperatures below 450°C include aluminium nitride (AIN), zinc oxide (ZnO), and/or scandium aluminium nitride (ScAIN).

Some electronics (e.g., CMOS components) can be damaged at temperatures greater than 450°C. By using piezoelectric materials that are processable at a temperature below 450°C, MEMS and electronic components can be integrated whilst avoiding degradation of the electronics, thereby improving the operation and efficiency of the device.

In particular, the use of ScAIN facilitates the use of low powers to operate the microneedle device, thereby improving energy efficiency, portability, and safety.

According to another aspect of the invention, there is provided a valve comprising:
a substrate;
a piezoelectric actuator formed on the substrate, wherein the piezoelectric actuator comprises a moveable piezoelectric diaphragm;
control circuitry integrally formed with the substrate for controlling the movement of the piezoelectric actuator;
the valve having a first state for allowing fluid flow therethrough, wherein in the first state the moveable piezoelectric diaphragm is deformed in a first sense; and
the valve having a second state for stopping fluid flow therethrough, wherein in the second state the moveable piezoelectric diaphragm is deformed in a second sense.

According to another aspect of the invention, there is provided a wearable patch comprising any of the microneedle devices or valves described herein.

According to another aspect of the invention, there is provided a liquid suitable for use with any of the microneedle devices, valves or patches described herein.

It will be understood that liquids will typically require specific properties to be suitable to be used with the microneedle device described herein. In particular, the rheological properties must not be such that, for liquids comprising medically active components, damage is caused to the medically active components that inhibits or even substantially prevents the desired medical effect.

### Description of the Drawings

An example embodiment of the present invention will now be illustrated with reference to the following Figures in which:
Figures 1-2 are schematic representations of a microneedle device, according to the invention;
Figure 3 is a schematic representation of part of a microneedle device according to the invention, showing a PMUT element in more detail;
Figure 4 is a schematic representation of a microneedle array according to the invention;
Figure 5 is a schematic representation of a microneedle device according to the invention;
Figure 6A is a schematic representation of a microneedle device having a valve arrangement in a first configuration, according to the invention, and
Figure 6B is a schematic representation of the same microneedle device as Figure 6A showing the valve arrangement in a second configuration, according to the invention.

### Detailed Description of an Example Embodiment

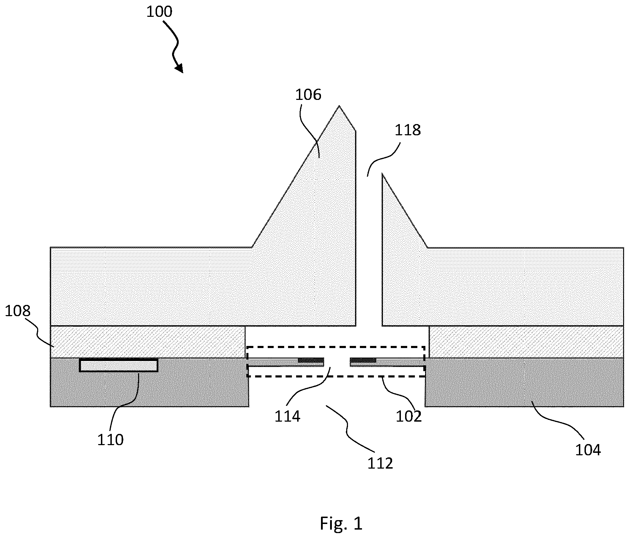

Figure 1 is a schematic representation of a microneedle device 100 having a PMUT element 102 formed on a substrate 104. One microneedle 106 extends from the substrate 104 above the PMUT element 102. An outlet valve seat 108 is provided between the microneedle 106 and the substrate 104. CMOS control circuitry 110 comprising a plurality of transistors is integrally formed with the substrate 104, and electrically couped to the PMUT element 102 via conductive connections (not shown). A cavity 112 is defined in part by the moveable piezoelectric diaphragm 102 and in part by the substrate 104.

In use, fluid flows from the cavity 112 via the aperture 114 through the centre of the moveable piezoelectric diaphragm 102. The fluid is delivered to the user via a channel 118.

Further, in use, the moveable piezoelectric diaphragm of the PMUT element 102 is configured to deform into the cavity 112. The CMOS control circuitry 110 is configured to receive electrical signals generated by the movement of the moveable piezoelectric diaphragm 102. According, the PMUT element 102 together with the CMOS control circuitry 110 operates as a sensor to allow the status of the microneedle 106 to be sensed. The control circuitry 110 generates a signal which can be used to determine multiple operational conditions - e.g., whether the microneedle 106 is correctly inserted in a user's skin or whether the microneedle 106 is blocked by an air bubble. In this embodiment, the operational conditions are determined based on the frequency of oscillation of the membrane when in contact with the fluid. The inventors have realised that the frequency of oscillation is affected by air bubbles blocking the channel 118 and the insertion status of the microneedle 106, for example. In other embodiments, the act of inserting the microneedle 106 into the user's skin may cause a force to be transferred through fluid (between the microneedle 106 and the PMUT element 102) and exerted on the moveable piezoelectric membrane. The resultant movement of the moveable piezoelectric membrane may be detected and used to identify correct insertion of the microneedle 106. In other embodiments, the PMUT element 102 may be used to transmit ultrasound and receive a reflected signal. The received signal may be used to determine correct insertion of the microneedle 106/presence of a blockage in the channel 118.

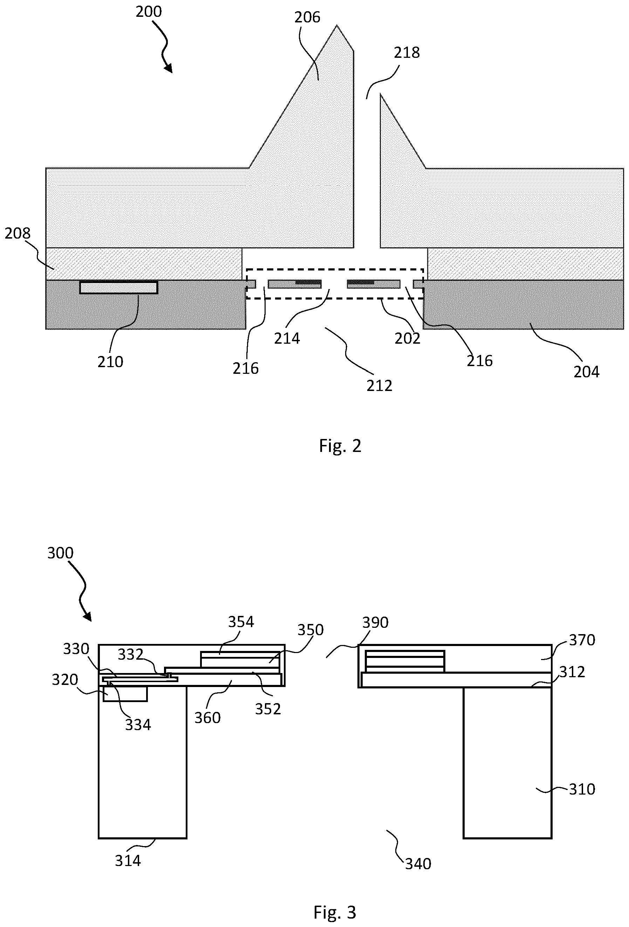

Figure 2 shows another schematic representation of a microneedle device 200 having a PMUT element 202 formed on a substrate 204.

The microneedle device 200 is similar to the microneedle device 100 shown in Figure 1. In particular, the microneedle device 200 also comprises a microneedle 206 having a channel 218 therethrough, an outlet valve seat 208, CMOS control circuitry 210 and a cavity 212. One difference between the embodiment shown in Figure 1 and the embodiment shown in Figure 2, is that the PMUT element 202 has a central aperture 214 through the moveable piezoelectric membrane in addition to peripheral apertures 216.

In the embodiment shown at Figure 2, the CMOS control circuitry 210 is configured to control movement of the moveable piezoelectric diaphragm of the PMUT element 202 to force fluid through the channel 218 and, in a different operational mode, to extra fluid from a target body through the channel 218. That is, in use, the PMUT element 202 functions as a pump for delivery and extraction of fluid through the microneedle 206 coupled therewith. In this embodiment, the CMOS control circuitry is also configured to provide sensing capabilities as described in relation to Figure 1.

Figure 3 shows one possible configuration of part of a microneedle device 300. The device 300 comprises a semiconductor substrate 310 having a first surface 312 opposite to a second surface 314. The device 300 includes CMOS control circuitry comprising a plurality of transistors 320 integrally provided within the substrate 310. A CMOS metallisation layer 330 is provided on the first surface 312. The PMUT element comprises a moveable piezoelectric diaphragm including a piezoelectric body 350, a first electrode 352 and a second electrode 354 each arranged on the first surface 312.

The moveable piezoelectric diaphragm also includes an additional layer 360 (made from the same material as the rest of the substrate, in this embodiment). A cavity 340 is defined in part by the moveable piezoelectric diaphragm and in part by the semiconductor substrate 310. In this embodiment, the CMOS metallisation layer 330 includes conductive connections 332, 334 extending from the plurality of transistors 320 to the first electrode 352. The device also includes a protective layer 370. In this embodiment, the moveable piezoelectric diaphragm does not extend across the full side of the cavity (i.e., it is discontinuous). Each of the first electrode 352, the second electrode 354, and the piezoelectric body 350 are annular and arranged concentrically. The additional layer 360 and protective layer 370 are also discontinuous such that an aperture 390 is defined through the moveable piezoelectric diaphragm - thereby increasing the flexibility of the piezoelectric diaphragm and allowing the flow of fluid therethrough.

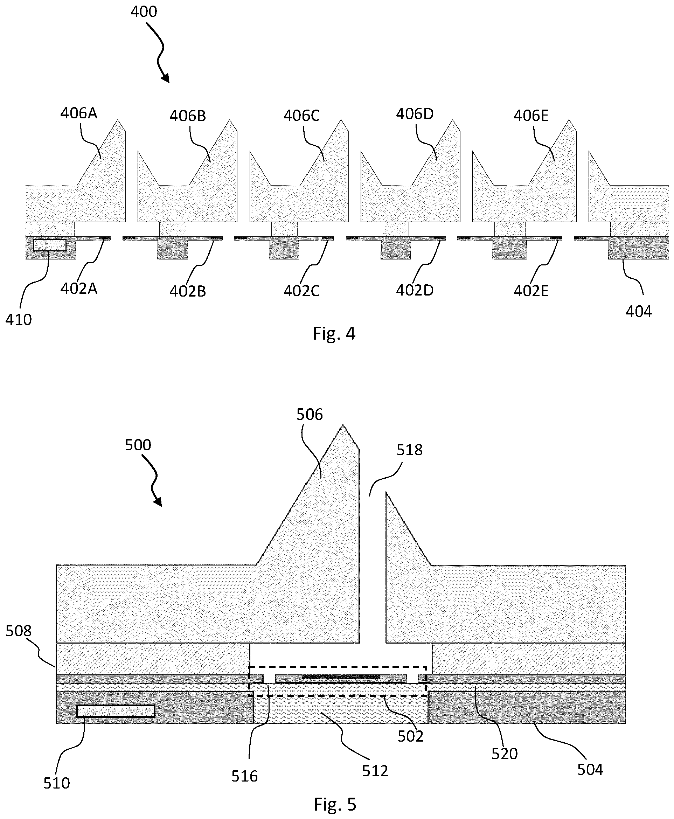

Figure 4 shows a microneedle array 400 having a plurality of microneedles 406A-E extending from a substrate 404. Each of the microneedles 406A-E is coupled to a respective PMUT element 402A-E. Control circuitry 410 is provided in the substrate 404 and coupled to each of PMUT elements 402A-E. Accordingly it is possible to provide sensing/pumping capabilities on the level of individual microneedles, whilst still providing a compact microneedle array.

Figure 5 shows another schematic representation of a microneedle device 500. The microneedle device has a PMUT element 502, having peripheral apertures 516 for allowing the flow of a fluid therethrough. The microneedle device 500 further comprises a microneedle 506, having a channel 518, extending from a substrate 504. The microneedle device 500 comprises an outlet valve seat 508, control circuitry 510 and a cavity 512. In this embodiment the cavity 512 is filled with a pharmaceutical fluid. The microneedle device 500 further comprises a channel extending parallel to the PMUT element 502 for directing the pharmaceutical fluid into the cavity 512 from a reservoir (not shown).

In use, the microneedle 506 pierces a user's skin. Pharmaceutical fluid can then flow from the cavity 512 through the apertures 516 into the user's skin. In this embodiment, the control circuitry 510 is configured to control movement of the moveable piezoelectric diaphragm 502 to transmit ultrasound towards the microneedle. This transmitted ultrasound can help to drive the pharmaceutical fluid through the channel 518 and enhance drug absorption by increasing the permeability of the skin.

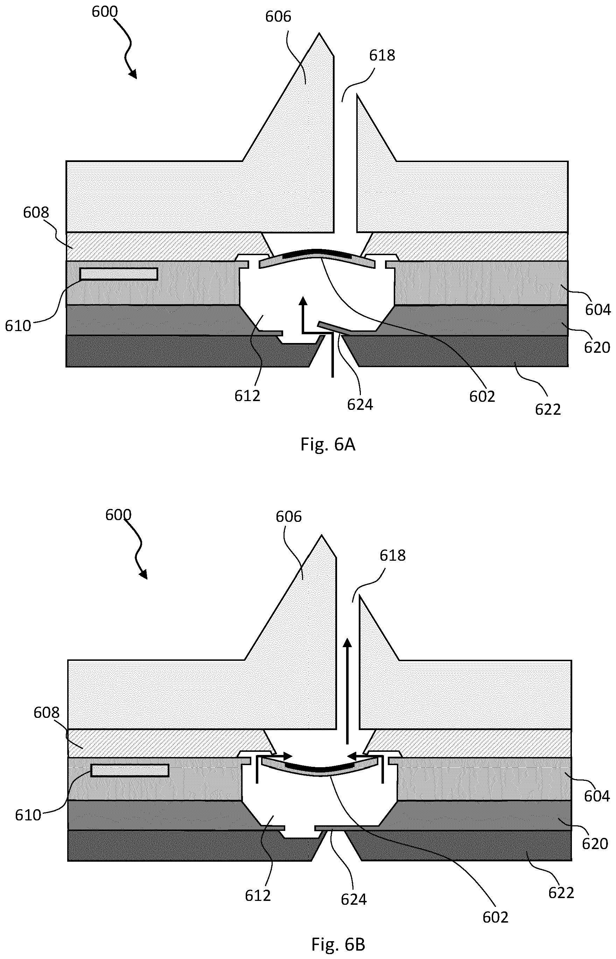

Figures 6A and 6B show schematic representations of a microneedle device 600 having a valve arrangement. The microneedle device 600 has a PMUT element 602 formed on a substrate 604. A microneedle 606 has a channel 618 and extends from the substrate 604. The microneedle device 600 comprises an outlet valve seat 608, a check valve 620, an inlet valve seat 622, control circuitry 610, a cavity 612 and a passive valve 624.

Figure 6A shows the microneedle device 600 in a first configuration. In this configuration the moveable piezoelectric diaphragm of the PMUT element 602 is deformed in a first sense. This movement is controlled by the control circuitry 610. This causes the valve 624 to open and for fluid to flow into the cavity 612. In the first configuration, fluid cannot flow through the PMUT element 602 to access the channel 618 of the microneedle 600.

Figure 6B shows the microneedle device 600 in a second configuration. In this configuration the moveable piezoelectric diaphragm of the PMUT element 602 is deformed in a second sense. This causes the valve 624 to close. In the second configuration, fluid can flow through the PMUT element 602 to access the channel 618 of the microneedle 600.

That is, in this embodiment, the PMUT element 602 operates as a valve.

Manufacturing the microneedle devices described herein typically involves a step of forming each required cavity within a substrate. Typically, the cavities are provided using a DRIE etch procedure from the backside of the substrate. Buried oxide and deep trench isolation structures can be used to define the cavity dimensions. The control circuitry can then be formed within the substrate. For example, transistors may be formed by standard CMOS processing methodologies including ion implantation on a p-type or n-type substrate. Metallisation layers can also formed by standard processes such as ion implantation chemical vapour deposition, physical vapour deposition, etching, chemical-mechanical planarization and/or electroplating.

Each moveable piezoelectric diaphragm can then be formed on the substrate. Forming each moveable piezoelectric diaphragms involves forming the respective electrodes and intervening piezoelectric body. Typically this step involves using successive thin film deposition techniques. Preferably, each piezoelectric body is formed of a material such as AIN or ScAIN which may be deposited at a temperature below 450°C by physical vapour deposition (including low-temperature sputtering). The electrodes are formed of, for example titanium, platinum, aluminium, tungsten or alloys thereof.

Each microneedle (or group of microneedles) can then be coupled to substrate (optionally via another layer).

Throughout the description and claims of this specification, the words "comprise" and "contain" and variations of them mean "including but not limited to", and they are not intended to and do not exclude other components, integers, or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, or groups described in conjunction with a particular aspect, embodiment, or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

## Claims

1. A microneedle device comprising:
a substrate;
one or more microneedles extending from the substrate, and for insertion into a target body;
one or more piezoelectric micromachined ultrasound transducer, PMUT, elements formed on the substrate, wherein each of the one or more PMUT elements comprises a moveable piezoelectric diaphragm;
and control circuitry integrally formed with the substrate and electrically coupled to the one or more PMUT elements.

2. The microneedle device according to claim 1, wherein each of the one or more PMUT elements is coupled to at least one microneedle of the one or more microneedles, and optionally wherein each microneedle is coupled to a single PMUT element, and further optionally wherein each PMUT element is coupled to a corresponding single microneedle.

3. The microneedle device according to any preceding claim, wherein the control circuity is configured to receive electrical signals generated by the movement of the or each moveable piezoelectric diaphragm, and generate a signal based on the movement of the or each moveable piezoelectric diaphragm.

4. The microneedle device according to claim 3, further comprising a first circuit, wherein the first circuit is configured to determine a microneedle characteristic based on the signal, and optionally wherein the microneedle characteristic is indicative of whether at least one of the one or more microneedles is correctly inserted in the target body; and/or
wherein the first circuit is configured to determine a target body characteristic based on the signal, and optionally wherein the target body characteristic is indicative of an internal pressure of the target body.

5. The microneedle device according to claim 4, wherein the microneedle characteristic and/or the target body characteristic is determined in dependence on an oscillation of at least one of the moveable piezoelectric diaphragms, and optionally in dependence on a frequency of the oscillation.

6. The microneedle device according to claim 4 or 5 wherein the first circuit is configured to generate an output signal based on the determined microneedle characteristic and/or target body characteristic, and optionally wherein the output signal is for disabling at least one of the one or more microneedles and/or for alerting the user that operation of the microneedle device is compromised.

7. The microneedle device according to any preceding claim, wherein the microneedle device is configured to extract liquid from the target body and/or deliver liquid to the target body, and optionally wherein the fluid is extracellular fluid or a pharmaceutical liquid.

8. The microneedle device according to claim 7 when dependent on claim 4 or 5, wherein, in use, each microneedle defines at least in part a respective fluid channel extending therethrough,
wherein the microneedle characteristic is indicative of whether a bubble exists within the fluid channel of a respective microneedle, and optionally,
wherein the first circuit is configured to generate an output signal based on the microneedle characteristic, and further optionally,
wherein the output signal is for triggering removal of the bubble, disabling a microneedle and/or for alerting the user that operation of the microneedle device is compromised.

9. The microneedle device according to any of claims 4-6 or 8, wherein the control circuitry comprises the first circuit.

10. The microneedle device according to any preceding claim wherein the control circuitry is configured to control ultrasound transmission by controlling movement of the or each moveable piezoelectric diaphragm and/or control ultrasound reception by receiving signals resulting from movement of the or each moveable piezoelectric diaphragm, and/or
wherein the microneedle device further comprises a reservoir for storing a liquid therein, and optionally wherein the reservoir contains a liquid for delivery to the target body and/or a liquid extracted from the target body.

11. The microneedle device according to any preceding claim, wherein the control circuitry is configured to control movement of the or each moveable piezoelectric diaphragm to extract liquid from the target body and/or deliver liquid to the target body, and optionally
wherein the microneedle device further comprises a temperature sensor for measuring the liquid temperature, wherein the control circuitry is configured to control movement of the or each moveable piezoelectric diaphragm based on the liquid temperature.

12. The microneedle device according to any preceding claim, the substrate having a first surface, the one or more microneedles extending from the first surface and the one or more PMUT elements formed on the first surface, wherein at least a portion of the control circuitry is integrally formed at the first surface, and/or
wherein the one or more PM UT elements each comprise one or more materials processable at a temperature below 450°C, and optionally wherein the or each moveable piezoelectric diaphragm comprises scandium aluminium nitride.

13. A valve comprising:
a substrate;
a piezoelectric actuator formed on the substrate, wherein the piezoelectric actuator comprises a moveable piezoelectric diaphragm;
control circuitry integrally formed with the substrate for controlling the movement of the piezoelectric actuator;
the valve having a first state for allowing fluid flow therethrough, wherein in the first state the moveable piezoelectric diaphragm is deformed in a first sense; and
the valve having a second state for stopping fluid flow therethrough, wherein in the second state the moveable piezoelectric diaphragm is deformed in a second sense.

14. A wearable patch comprising the microneedle device according to any of claims 1-12 or the valve according to claim 13.

15. A liquid suitable for use with the microneedle device according to any of claims 1-12, the valve according to claim 13, or the patch according to claim 14.
